(19) Europäisches Patentamt
European Patent Office
Office européen des brevets

(11) Veröffentlichungsnummer: **0 237 630 B1**

(12) **EUROPÄISCHE PATENTSCHRIFT**

(45) Veröffentlichungstag der Patentschrift: **15.07.92**

(51) Int. Cl.[5]: **C07D 233/38**

(21) Anmeldenummer: **86116149.5**

(22) Anmeldetag: **21.11.86**

(54) **Verfahren zur Herstellung chiraler Glycinderivate.**

(30) Priorität: **14.02.86 DE 3604591**

(43) Veröffentlichungstag der Anmeldung:
**23.09.87 Patentblatt 87/39**

(45) Bekanntmachung des Hinweises auf die Patenterteilung:
**15.07.92 Patentblatt 92/29**

(84) Benannte Vertragsstaaten:
**AT BE CH DE ES FR GB GR IT LI LU NL SE**

(56) Entgegenhaltungen:
**DE-A- 3 334 855**

(73) Patentinhaber: **Degussa Aktiengesellschaft**
**Weissfrauenstrasse 9**
**W-6000 Frankfurt am Main 1(DE)**

(72) Erfinder: **Seebach, Dieter, Dr.**
**Orellistrasse 3**
**CH-8044 Zürich(CH)**
Erfinder: **Fitzi, Robert**
**Carl-Spitteler-Strasse 53**
**CH-8053 Zürich(CH)**

EP 0 237 630 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von (R)- und (S)-1,3-Imidazolidin-4-onen der allgemeinen Formel

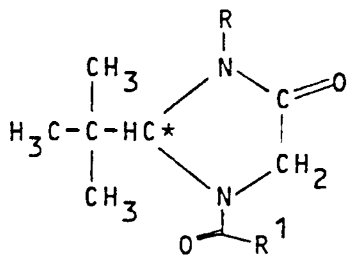

(I),

in der * ein Asymetriezentrum darstellt.

Bisher waren die chiralen 1,3-Imidazolidin-4-one der allgemeinen Formel (I) nur durch Abbaureaktionen von in 5-Stellung substituierten 1,3-Imidazolidin-4-onen zugänglich, die über mehrstufige Synthesen ausgehend von L- bzw. D-Methionin bzw. L- bzw. D-O-Benzylserin erhalten wurden (D. Seebach, D.D. Miller, S. Müller und T. Weber, Helv. Chim. Acta 68 , 949 (1985)).

Aufgabe der Erfindung ist ein Verfahren zur Herstellung von Verbindungen gemäß Formel (I), bei dem man mit billigen, achiralen Ausgangsprodukten arbeiten kann.

Gegenstand der Erfindung ist ein Verfahren zur Herstellung von (R)- und (S)-1,3-Imidazolidin-4-onen der allgemeinen Formel

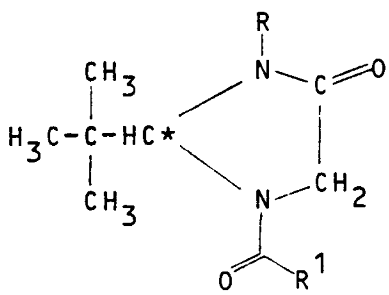

(I),

in der * ein Asymetriezentrum, R eine geradkettige oder verzweigte $C_1$- bis $C_4$-Alkylgruppe und $R^1$ eine Phenyl-oder Benzylgruppe, Phenyl- oder Benzyloxygruppe, bevorzugt die Phenylgruppe oder eine in beliebiger Ringstellung 1- bis 3-fach durch eine $C_1$-$C_4$-Alkyl- oder Alkoxygruppe substituierte Phenylgruppe bedeuten, welches dadurch gekennzeichnet ist, daß man ein racemisches 1,3-Imidazolidin-4-on der allgemeinen Formel

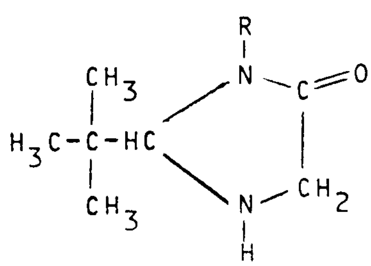

(II),

in der R die bereits angegebene Bedeutung hat, durch eine Umsetzung mit einer chiralen Säure der allgemeinen Formel

$*R^2 - COOH$ (IV),

in der *$R^2$ einen chiralen Rest darstellt, in ein diasteromeres Salzpaar aus z.B. (S)-Säure/(S)-II und (S)-Säure/(R)-II überführt.

Als chirale Säuren lassen sich prinzipiell literaturbekannte Verbindungen wie N-Acetylaminosäuren, Z-geschützte Aminosäuren, Pyroglutaminsäure, Weinsäure, Äpfelsäure, Campher-10-sulfonsäure, Dibenzoylweinsäure und Deoxycholsäure, bevorzugt jedoch (R)- bzw. (S)-Mandelsäure und (-)-Diaceton-2-ketogulonsäure einsetzen.

Dies erfolgt bei Temperaturen von -30 °C bis 25 °C aus Lösungen von Verbindungen gemäß Formel (II) in Lösungsmitteln wie z.B. Alkoholen, insbesondere Methanol oder Ethanol, Gemischen dieser Alkohole mit Wasser, Essigester oder chlorierten Kohlenwasserstoffen, insbesondere Methylenchlorid oder Chloroform. Besonders geeignet ist Aceton.
Man wählt dabei jeweils das Lösungsmittel in Abhängigkeit von den zu trennenden Verbindungen so aus, daß die Löslichkeitsunterschiede zwischen beiden Salzpaaren groß sind. Beispielweise zeigen das Salz aus (-)-Diaceton-2-ketogulonsäure und (S)-II und das Salz aus (R)-Mandelsäure und (R)-II in Ethanol die größere Tendenz zum Auskristallisieren im Verhältnis zu ihren optischen Antipoden.
Nach der Trennung der beiden diastereomeren Salzpaare durch fraktionierte Kristallisation, der man gegebenenfalls zur Reinigung eine Umkristallisation anschließt, erhält man durch Umsetzung der vorzugsweise in einem chlorierten Kohlenwasserstoff, insbesondere Methylenchlorid oder Chloroform, suspendierten Salze mit wässriger Natron- oder Kalilauge, deren Menge geringfügig über der stöchiometrisch notwendigen liegt, die 1,3-Imidazolidin-4-one (R)-II bzw. (S)-II.

Anschließend werden die (R)- bzw. (S)-1,3-Imidazolidin-4-one der allgemeinen Formel (II) ohne Isolierung auf an sich bekannte Weise mit einer Verbindung der allgemeinen Formel (IIIa) oder (IIIb) in Gegenwart von Triethylamin, Pyridin oder Natron- bzw. Kalilauge zu den (R)- bzw. (S)-1,3-Imidazolidin-4-onen der allgemeinen Formel (I) umgesetzt (vgl. D. Seebach, D.D. Miller, S. Müller und T. Weber, Helv. Chim. Acta 68 , 949 (1985)).

Der jeweils andere optische Antipode ist nach Einengen der Kristallisationsmutterlauge auf analoge Weise zugänglich.
Nach mehrmaliger Umkristallisation und anschließender Trocknung bei vermindertem Druck bis zur Gewichtskonstanz erhält man so in guten chemischen Ausbeuten und in optischen Ausbeuten von 98 - 99% die kristallinen (R)- und (S)-1,3-Imidazolidin-4-one der allgemeinen Formel (I).
Bis zu 95% der eingesetzten chiralen Säure der allgemeinen Formel (IV) können ohne nachweisbare Racemisierung zurückgewonnen werden. Hierzu wird die wäßrige Phase nach Abtrennung von (R)- bzw. (S)-I mit Mineralsäuren wie Schwefelsäure oder Salzsäure angesäuert und mit einem mit Wasser nicht mischbaren Lösungsmittel wie z.B. Essigester extrahiert.

Das erfindungsgemäße Verfahren eröffnet einen hervorragenden Zugang zu enantiomerenreinen Glycinderivaten der allgemeinen Formel (I) aus achiralen Ausgangsprodukten und bildet damit, wie z.B von D. Seebach et al. in DE-OS 3 334 855 oder Helv. Chim. Acta 68 , 949 (1985) beschrieben, nach diastereoselektiver ein- oder zweimaliger $\alpha$-Alkylierung und Ringspaltung eine vorteilhafte Basis für die Synthese verzweigter und nicht verzweigter, proteinogener und nicht proteinogener (R)- und (S)-Aminosäuren.

Zur Durchführung des erfindungsgemäßen Verfahrens wird zunächst auf bekannte Weise aus einem Glycinamid der allgemeinen Formel

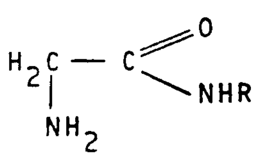

(V)

in der R die bereits angegebene Bedeutung hat und Pivalaldehyd das racemische 1,3-Imidazolidin-4-on der allgemeinen Formel (II) hergestellt (R. Naef und D. Seebach, Helv. Chim. Acta 68 , 135 (1985)). Ein alternatives Darstellungsverfahren ergibt sich aus dem von D. Seebach et al. in Helv. Chim. Acta 68 , 949 (1985) beschriebenen Abbau einer Seitenkette in 5-Stellung, ausgehend von DL-Aminosäuren wie Methionin oder O-Benzylserin.

## Beispiele:

Das erfindungsgemäße Verfahren wird durch die nachfolgenden Beispiele näher erläutert:

a) Herstellung von (R,S)-2-(t-Butyl)-3-methyl-1,3-imidazolidin-4-on

Die durch Zugabe von 125,6 g (1 Mol) Glycinmethylesterhydrochlorid zu eisgekühlten 375 ml 8M ethanolischem Methylamin erhaltene Suspension wurde 15 Stunden bei Raumtemperatur gerührt und unter vermindertem Druck zu einem zähen Brei eingeengt. Dieser wurde dreimal mit je 200 ml Methylenchlorid aufgeschlämmt und wieder eingeengt. Den Rückstand versetzte man mit 1 l Methylenchlorid, 235 ml (1,5 Mol) Pivalaldehyd und 209 ml (1,5 Mol) Triethylamin und kochte 10 Stunden am Wasserabscheider. Nach Filtration, Waschen des Rückstandes mit 500 ml Ether und Einengen der Filtrate unter vermindertem Druck wurde eine Lösung des erhaltenen Öls in 300 ml Methanol unter Eiskühlung mit 600 ml HCl-gesättigtem Methanol versetzt, 0,5 Stunden bei 0°C und 2 Stunden bei Raumtemperatur gerührt und wieder eingedampft. Eine Lösung des Sirups in 800 ml Methylenchlorid wurde unter Eiskühlung mit 670 ml 3M Natronlauge gewaschen und zu 107,5 g (69%) rohem (R,S)-2-(t-Butyl)-3-methyl-1,3-imidazolidin-4-on eingeengt, einem gelblichen Öl, das in der Kälte kristallisierte. Analytik siehe R.Naef und D. Seebach, Helv. Chim. Acta 68 , 135 (1985).

b) Enantiomerentrennung von (R,S)-2-(t-Butyl)-3-methyl-1,3-imidazolidin-4-on

Eine Mischung aus 70 g (0,448 Mol) des nach a) hergestellten (R,S)-2-(t-Butyl)-3-methyl-1,3-imidazolidin-4-ons und 70 g (R)-(-)-Mandelsäure wurde in siedendem Aceton gelöst. Nach Abkühlen auf Raumtemperatur innerhalb von 6 Stunden und weiteren 15 Stunden bei 5°C wurde der ausgefallene Kristallkuchen abfiltriert. Nach Trocknen wurden 54,5 g leicht gelbes Kristallisat aus (R)-Mandelsäure und (R)-2-(t-Butyl)-3-methyl-1.3-imidazolidin-4-on isoliert.

c) Herstellung von (S)-(+)-1-Benzoyl-2-(t-Butyl)-3-methyl-1,3-imidazolidin-4-on

Eine Suspension des nach b) erhaltenen Diastereomerensalzes aus (R)-(-)-Mandelsäure und (R)-2-(t-Butyl)-3-methyl-1,3-imidazolidin-4-on (54,5 g, 0,177 Mol) in 200 ml Methylenchlorid wurde mit 92 ml 2M Natronlauge versetzt und geschüttelt. Durch Extraktion der mit 50% iger Schwefelsäure angesäuerten Wasserphase mit 300 ml Essigsäureethylester können bis zu 25,5 g (R)-(-)-Mandelsäure zurückgewonnen werden.

Das in der orgsnischen Phase verbliebene 1,3-Imidazolidin-4-on wurde durch Zugabe von 20,1 ml (0,173 Mol) Benzoylchlorid und 195 ml 1M Natronlauge unter Eiskühlung benzoyliert. Die organische Phase wurde abgetrennt, uber Magnesiumsulfat getrocknet und unter vermindertem Druck eingeengt.
Man erhält 45,8 g gelbliches Kristallisat mit $[\alpha]_D = +107°$ (c=1, $CH_2Cl_2$).
Zweimalige Umkristallisation aus Ethanol, Fällung aus Methylenchlorid mit Pentan und 12 stündiges Trocknen bei 0.1 Torr und 60°C lieferte 34,7 g (60%) (S)-(+)-1-Benzoyl-2-(t-Butyl)-3-methyl-1,3-imidazolidin-4-on mit $[\alpha]_D = +127°$ (c=1, $CH_2Cl_2$).
Durch dreimalige Umkristallisation einer Probe aus Ethanol und anschließender Sublimation (135°C/0,01 Torr) erhielt man ein $[\alpha]_D$ von +127,5° und einen Schmelzpunkt von 143-144°C.

Die spektroskopischen Daten stimmen mit der von Seebach et al. in Helv. Chim. Acta 68 , 949 (1985) beschriebenen (R,S)-Verbindung überein.

d) Herstellung von (R)-(-)-1-Benzoyl-2-(t-Butyl)-3-methyl-1,3-imidazolidin-4-on

Der durch Einengen der nach b) erhaltenen Kristallisationsmutterlauge zurückbleibende Sirup wurde in 300 ml Methylenchlorid aufgenommen und dann - wie unter c) beschrieben - zuerst die Mandelsäure ausgewaschen (140 ml 2M Natronlauge) und dann das 1,3-Imidazolidin-4-on direkt in der organischen Phase benzoyliert (30 ml (0,258 Mol) Benzoylchlorid, 280 ml 1M Natronlauge).

Das beim Einengen der organischen Phase unter vermindertem Druck erhaltene Rohkristallisat wurde aus 45 ml Ethanol umkristallisiert.
Man erhielt 33,6 g Kristalle mit $[\alpha]_D = -109°$ (c=1, $CH_2Cl_2$).
Umkristallisation und Trocknung - wie unter c) beschrieben - lieferte 26,4 g (45%) (R)-(-)-1-Benzoyl-2-(t-Butyl)-3-methyl-1,3 imidazolidin-4-on mit $[\alpha]_D = -126°$ (c=1, $CH_2Cl_2$).

## Patentansprüche

1. Verfahren zur Herstellung enantiomerenreiner (R)- und (S)-1,3-Imidazolidin-4-one der allgemeinen Formel

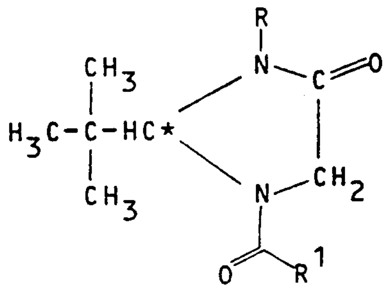

(I),

in der * ein Asymmetriezentrum, R eine geradkettige oder verzweigte $C_1$- bis $C_4$-Alkylgruppe und $R^1$ eine Phenyl-oder Benzylgruppe, Phenyl- oder Benzyloxygruppe oder eine in beliebiger Ringstellung 1- bis 3-fach durch eine $C_1$-$C_4$-Alkyl- oder Alkoxygruppe substituierte Phenylgruppe bedeuten, dadurch gekennzeichnet, daß man ein racemisches 1,3-Imidazolidin-4-on der allgemeinen Formel

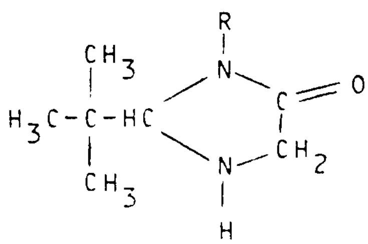

(II),

in der R die bereits angegebene Bedeutung hat, durch eine Umsetzung mit einer chiralen Säure in ein diastereomeres Salzpaar überführt, dieses fraktioniert kristallisiert, nach Auftrennung dieses Salzpaares (R)-II bzw. (S)-II isoliert und diese Verbindungen mit einem Säurehalogenid der allgemeinen Formel

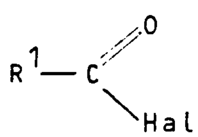

(IIIa),

Hal = Cl, Br

oder einem Anhydrid der allgemeinen Formel

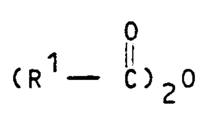

(IIIb),

in denen $R^1$ die bereits angegebene Bedeutung hat, umsetzt.

**2.** Verfahren gemäß Anspruch 1, dadurch gekennzeichnet, daß man (R,S)-2-(t-Butyl)-3-methyl-1,3-imidazolidin-4-on einsetzt.

**3.** Verfahren gemäß den Ansprüchen 1 und 2, dadurch gekennzeichnet, daß man als chirale Säure (R)- bzw. (S)-Mandelsäure verwendet.

4. Verfahren gemäß den Ansprüchen 1 und 2, dadurch gekennzeichnet, daß man als chirale Säure (-)-Diaceton-2-ketogulonsäure einsetzt.

**Claims**

1. A process for the preparation of enantiomerically pure (R)- and (S)-1,3-imidazolidin-4-ones corresponding to the general formula

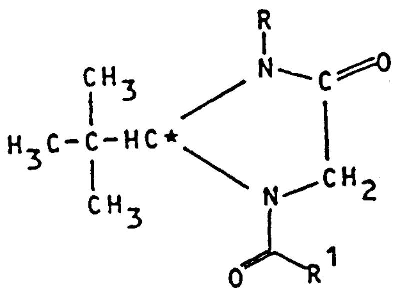

(I),

in which * denotes an asymmetric centre, R denotes a straight chained or branched $C_1$ to $C_4$ alkyl group and $R^1$ denotes a phenyl or benzyl or phenyloxy or benzyloxy group or a phenyl group which is mono- to tri-substituted by a $C_1$ to $C_4$ alkyl or alkoxy group in any ring position, characterised in that a racemic 1,3-imidazolidin-4-one corresponding to the general formula

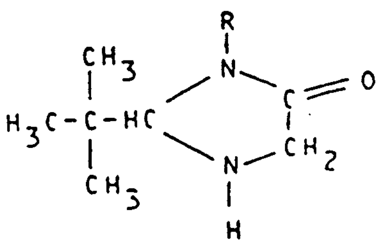

(II),

in which R has the meaning already indicated is converted into a diastereomeric salt pair by reaction with a chiral acid, the said salt pair is fractionally crystallised, (R)-II and (S)-II are isolated after separation of this salt pair, and these compounds are reacted with an acid halide corresponding to the general formula

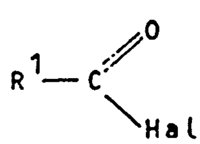

(IIIa),

Hal = Cl, Br

or an anhydride corresponding to the general formula

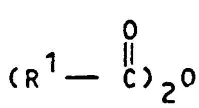

(IIIb),

in which $R^1$ has the meaning already indicated.

**2.** A process according to claim 1, characterised in that (R,S)-2-(t-butyl)-3-methyl-1,3-imidazolidin-4-one is used.

**3.** A process according to claims 1 and 2, characterised in that the chiral acid used is (R)- or (S)-mandelic acid.

**4.** A process according to claims 1 and 2, characterised in that the chiral acid used is (-)-diacetone-2-ketogluconic acid.

**Revendications**

**1.** Procédé pour la préparation de (R)- et (S)-1,3-imidazolidine-4-ones sous forme d'énantiomères purs, de formule générale

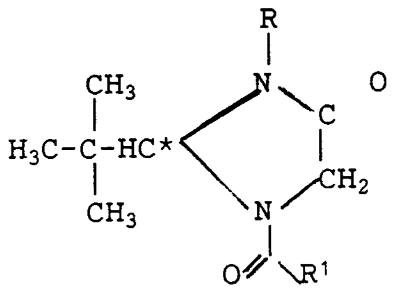

(I)

dans laquelle * signifie un centre d'asymétrie, R représente un groupe alkyle en $C_1$-$C_4$ à chaîne droite ou ramifiée, et $R^1$ représente un groupe phényle ou benzyle, un groupe phényloxy ou benzyloxy ou un groupe phenyle substitué de 1 à 3 fois, en position(s) quelconque(s) sur le noyau, par un (des) groupe-(s) alkyle ou alcoxy en $C_1$-$C_4$,
caractérisé en ce que l'on convertit une 1,3-imidazolidine-4-one racémique de formule générale.

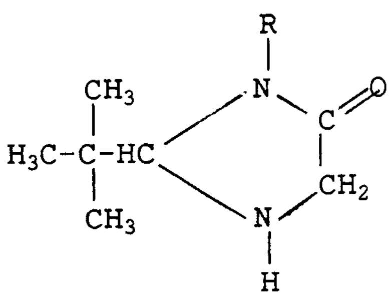

(II)

dans laquelle R a la signification déjà indiquée, par une réaction avec un acide chiral, en une paire de sels diastéréoisomères, on soumet celle-ci à une cristallisation fractionnée, après résolution de cette paire de sels on isole (R)-II et (S)-II, respectivement et on fait réagir ces composés avec un halogénure d'acide de formule générale.

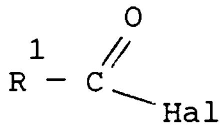 (IIIa),

Hal = CL, Br

ou avec un anhydride de formule générale

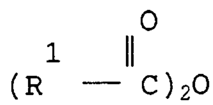 (IIIb),

formules dans lesquelles $R^1$ à la signification déjà donnée.

2. Procédé selon la revendication 1, caractérisé en ce que l'on met en réaction la (R, S)-2-(tert-butyl)-3-méthyl-1,3-imidazolidine-4-one.

3. Procédé selon les revendications 1 et 2, caractérisé en ce que l'on utilise, en tant qu'acide chiral, l'acide (R)- ou (S)-mandélique.

4. Procédé selon les revendications 1 et 2, caractérisé en ce que l'on utilise, en tant qu'acide chiral, l'acide (-)-diacétone-2-cétogulonique.